# EUROPEAN PATENT SPECIFICATION

(11) **EP 3 067 697 B1**
(45) Date of publication and mention of the grant of the patent: **08.05.2019**
(21) Application number: 14859384.1
(22) Date of filing: 05.11.2014
(51) Int. Cl.: C07K 14/005, G01N 33/543, G01N 33/53, G01N 33/569

(54) **METHOD FOR REDUCING NON-SPECIFIC REACTIONS IN IMMUNOASSAY OF ANTI-HTLV ANTIBODIES**
VERFAHREN ZUR REDUZIERUNG NICHTSPEZIFISCHER REAKTIONEN BEI EINEM IMMUNOASSAY FÜR ANTI-HTLV-ANTIKÖRPER
PROCÉDÉ DE RÉDUCTION DES RÉACTIONS NON-SPÉCIFIQUES DANS UN DOSAGE IMMUNOLOGIQUE D'ANTICORPS ANTI-HTLV

(30) Priority: 06.11.2013 JP 2013229924
(43) Date of publication of application: 14.09.2016
(73) Proprietor: Fujirebio Inc., Tokyo 163-0410 (JP)
(72) Inventor: MIYAGAWA, Eiji, Tokyo 163-0410 (JP); TAKAHASHI, Kazuya, Tokyo 163-0410 (JP); TANIMOTO, Tetsuji, Tokyo 163-0410 (JP)
(74) Representative: Mewburn Ellis LLP
(86) International application number: PCT/JP2014/079301
(87) International publication number: WO 2015/068715

(56) References cited:
- JP-A- H05 508 838
- JP-A- 2000 078 973
- JP-A- 2008 249 603
- H.C. Li: "Q2VA24_9DELA", , 16 October 2013 (2013-10-16), XP055364436, Retrieved from the Internet: URL:http://www.uniprot.org/uniprot/Q2VA24. txt?version=31 [retrieved on 2017-04-12]
- R Mahieux ET AL: "Molecular characterization and phylogenetic analyses of a new, highly divergent simian T-cell lymphotropic virus type 1 (STLV-1marc1) in Macaca arctoides", Journal of Virology, 1 August 1997 (1997-08-01), pages 6253-6258, XP055364220, UNITED STATES Retrieved from the Internet: URL:http://jvi.asm.org/content/71/8/6253.f ull.pdf -& R. Mahieux: "Q9IZL0_9STL1", , 16 October 2013 (2013-10-16), pages 1-1, XP055364371, Retrieved from the Internet: URL:http://www.uniprot.org/uniprot/Q9IZL0. txt?version=31 [retrieved on 2017-04-12]
- LE BLANC I ET AL.: 'The PPPY motif of human T- cell leukemia virus type 1 Gag protein is required early in the budding process' J VIROL vol. 6, no. 19, pages 10024 - 10029, XP055342447

### SEQUENCE LISTING

<110> FUJIREBIO Inc.
<120> Method for reducing nonspecific reaction in immunoassay of HTLV antibody
<130> PF525-PCT
<160> 27
<170> PatentIn version 3.5
<210> 1
   <211> 28
   <212> PRT
   <213> Human T-cell lymphotropic virus
<400> 1
   Pro Ser Ser Pro Thr His Asp Pro Pro Asp Ser Asp Pro Gln Ile Pro
   1               5                   10                  15
   Pro Pro Tyr Val Glu Pro Thr Ala Pro Gln Val Leu
               20                  25
<210> 2
   <211> 28
   <212> PRT
   <213> Artificial Sequence
<220>
   <223> altered p19 peptide, No. 34
<400> 2
   Pro Ser Ser Pro Thr His Asp Pro Pro Ala Ser Asp Pro Gln Ile Pro
   1               5                   10                  15
   Pro Pro Tyr Val Glu Pro Thr Ala Pro Gln Val Leu
               20                  25
<210> 3
   <211> 28
   <212> PRT
   <213> Artificial Sequence
<220>
   <223> altered p19 peptide, No. 35
<400> 3
   Pro Ser Ser Pro Thr His Asp Pro Pro Asp Ala Asp Pro Gln Ile Pro
   1               5                   10                  15
   Pro Pro Tyr Val Glu Pro Thr Ala Pro Gln Val Leu
               20                  25
<210> 4
   <211> 28
   <212> PRT
   <213> Artificial Sequence
<220>
   <223> altered p19 peptide, No. 36
<400> 4
   Pro Ser Ser Pro Thr His Asp Pro Pro Asp Ser Ala Pro Gln Ile Pro
   1               5                   10                  15
   Pro Pro Tyr Val Glu Pro Thr Ala Pro Gln Val Leu
               20                  25
<210> 5
   <211> 28
   <212> PRT
   <213> Artificial Sequence
<220>
   <223> altered p19 peptide, No. 37
<400> 5
   Pro Ser Ser Pro Thr His Asp Pro Pro Asp Ser Asp Ala Gln Ile Pro
   1               5                   10                  15
   Pro Pro Tyr Val Glu Pro Thr Ala Pro Gln Val Leu
               20                  25
<210> 6
   <211> 28
   <212> PRT
   <213> Artificial Sequence
<220>
   <223> altered p19 peptide, No. 38
<400> 6
   Pro Ser Ser Pro Thr His Asp Pro Pro Asp Ser Asp Pro Ala Ile Pro
   1               5                   10                  15
   Pro Pro Tyr Val Glu Pro Thr Ala Pro Gln Val Leu
               20                  25
<210> 7
   <211> 28
   <212> PRT
   <213> Artificial Sequence
<220>
   <223> altered p19 peptide, No. 39
<400> 7
   Pro Ser Ser Pro Thr His Asp Pro Pro Asp Ser Asp Pro Gln Ala Pro
   1               5                   10                  15
   Pro Pro Tyr Val Glu Pro Thr Ala Pro Gln Val Leu
               20                  25
<210> 8
   <211> 28
   <212> PRT
   <213> Artificial Sequence
<220>
   <223> altered p19 peptide, No. 40
<400> 8
   Pro Ser Ser Pro Thr His Asp Pro Pro Asp Ser Asp Pro Gln Ile Ala
   1               5                   10                  15
   Pro Pro Tyr Val Glu Pro Thr Ala Pro Gln Val Leu
               20                  25
<210> 9
   <211> 28
   <212> PRT
   <213> Artificial Sequence
<220>
   <223> altered p19 peptide, No. 41
<400> 9
   Pro Ser Ser Pro Thr His Asp Pro Pro Asp Ser Ala Pro Gln Ala Pro
   1               5                   10                  15
   Pro Pro Tyr Val Glu Pro Thr Ala Pro Gln Val Leu
               20                  25
<210> 10
   <211> 28
   <212> PRT
   <213> Artificial Sequence
<220>
   <223> altered p19 peptide, No. 42
<400> 10
   Pro Ser Ser Pro Thr His Asp Pro Pro Asp Ser Asn Pro Gln Ile Pro
   1               5                   10                  15
   Pro Pro Tyr Val Glu Pro Thr Ala Pro Gln Val Leu
               20                  25
<210> 11
   <211> 28
   <212> PRT
   <213> Artificial Sequence
<220>
   <223> altered p19 peptide, No. 43
<400> 11
   Pro Ser Ser Pro Thr His Asp Pro Pro Asp Ser Asp Pro Gln Leu Pro
   1               5                   10                  15
   Pro Pro Tyr Val Glu Pro Thr Ala Pro Gln Val Leu
               20                  25
<210> 12
   <211> 28
   <212> PRT
   <213> Artificial Sequence
<220>
   <223> altered p19 peptide, No. 44
<400> 12
   Pro Ser Ser Pro Thr His Asp Pro Pro Asp Ser Asn Pro Gln Leu Pro
   1               5                   10                  15
   Pro Pro Tyr Val Glu Pro Thr Ala Pro Gln Val Leu
               20                  25
<210> 13
   <211> 28
   <212> PRT
   <213> Artificial Sequence
<220>
   <223> altered p19 peptide, No. 45
<400> 13
   Pro Ser Ser Pro Thr His Asp Pro Pro Asp Ser Glu Pro Gln Ile Pro
   1 5 10 15
   Pro Pro Tyr Val Glu Pro Thr Ala Pro Gln Val Leu
   20 25
<210> 14
   <211> 28
   <212> PRT
   <213> Artificial Sequence
<220>
   <223> altered p19 peptide, No. 46
<400> 14
   Pro Ser Ser Pro Thr His Asp Pro Pro Asp Ser Asn Pro Gln Glu Pro
   1 5 10 15
   Pro Pro Tyr Val Glu Pro Thr Ala Pro Gln Val Leu
   20 25
<210> 15
   <211> 28
   <212> PRT
   <213> Artificial Sequence
<220>
   <223> altered p19 peptide, No. 47
<400> 15
   Pro Ser Ser Pro Thr His Asp Pro Pro Asp Ser Glu Pro Gln Glu Pro
   1 5 10 15
   Pro Pro Tyr Val Glu Pro Thr Ala Pro Gln Val Leu
   20 25
<210> 16
   <211> 29
   <212> PRT
   <213> Artificial Sequence
<220>
   <223> K + Peptide No. 7
<400> 16
   Lys Pro Ser Ser Pro Thr His Asp Pro Pro Asp Ser Asp Pro Gln Ile
   1 5 10 15
   Pro Pro Pro Tyr Val Glu Pro Thr Ala Pro Gln Val Leu
   20 25
<210> 17
   <211> 29
   <212> PRT
   <213> Artificial Sequence
<220>
   <223> K + Peptide No. 36
<400> 17
   Lys Pro Ser Ser Pro Thr His Asp Pro Pro Asp Ser Ala Pro Gln Ile
   1               5                   10                  15
   Pro Pro Pro Tyr Val Glu Pro Thr Ala Pro Gln Val Leu
               20                  25
<210> 18
   <211> 29
   <212> PRT
   <213> Artificial Sequence
<220>
   <223> K + Peptide No. 39
<400> 18
   Lys Pro Ser Ser Pro Thr His Asp Pro Pro Asp Ser Asp Pro Gln Ala
   1               5                   10                  15
   Pro Pro Pro Tyr Val Glu Pro Thr Ala Pro Gln Val Leu
               20                  25
<210> 19
   <211> 29
   <212> PRT
   <213> Artificial Sequence
<220>
   <223> K + Peptide 41
<400> 19
   Lys Pro Ser Ser Pro Thr His Asp Pro Pro Asp Ser Ala Pro Gln Ala
   1               5                   10                  15
   Pro Pro Pro Tyr Val Glu Pro Thr Ala Pro Gln Val Leu
               20                  25
<210> 20
   <211> 29
   <212> PRT
   <213> Artificial Sequence
<220>
   <223> K + Peptide 42
<400> 20
   Lys Pro Ser Ser Pro Thr His Asp Pro Pro Asp Ser Asn Pro Gln Ile
   1               5                   10                  15
   Pro Pro Pro Tyr Val Glu Pro Thr Ala Pro Gln Val Leu
               20                  25
<210> 21
   <211> 29
   <212> PRT
   <213> Artificial Sequence
<220>
   <223> K + Peptide No. 43
<400> 21
   Lys Pro Ser Ser Pro Thr His Asp Pro Pro Asp Ser Asp Pro Gln Leu
   1               5                   10                  15
   Pro Pro Pro Tyr Val Glu Pro Thr Ala Pro Gln Val Leu
               20                  25
<210> 22
   <211> 29
   <212> PRT
   <213> Artificial Sequence
<220>
   <223> K + Peptide 44
<400> 22
   Lys Pro Ser Ser Pro Thr His Asp Pro Pro Asp Ser Asn Pro Gln Leu
   1               5                   10                  15
   Pro Pro Pro Tyr Val Glu Pro Thr Ala Pro Gln Val Leu
               20                  25
<210> 23
   <211> 29
   <212> PRT
   <213> Artificial Sequence
<220>
   <223> K + Peptide No. 45
<400> 23
   Lys Pro Ser Ser Pro Thr His Asp Pro Pro Asp Ser Glu Pro Gln Ile
   1 5 10 15
   Pro Pro Pro Tyr Val Glu Pro Thr Ala Pro Gln Val Leu
   20 25
<210> 24
   <211> 29
   <212> PRT
   <213> Artificial Sequence
<220>
   <223> K + Peptide No. 46
<400> 24
   Lys Pro Ser Ser Pro Thr His Asp Pro Pro Asp Ser Asn Pro Gln Glu
   1 5 10 15
   Pro Pro Pro Tyr Val Glu Pro Thr Ala Pro Gln Val Leu
   20 25
<210> 25
   <211> 29
   <212> PRT
   <213> Artificial Sequence
<220>
   <223> K + Peptide 47
<400> 25
   Lys Pro Ser Ser Pro Thr His Asp Pro Pro Asp Ser Glu Pro Gln Glu
   1 5 10 15
   Pro Pro Pro Tyr Val Glu Pro Thr Ala Pro Gln Val Leu
   20 25
<210> 26
   <211> 609
   <212> DNA
   <213> Human T-cell lymphotropic virus
<220>
   <221> CDS
   <222> (1)..(609)
<400> 26
   atg ggc caa atc ttt tcc cgt agc gct agc cct att ccg cga ccg ccc      48
   Met Gly Gln Ile Phe Ser Arg Ser Ala Ser Pro Ile Pro Arg Pro Pro
   1               5                   10                  15
   cgg ggg ctg gcc gct cat cac tgg ctt aac ttc ctc cag gcg gca tat      96
   Arg Gly Leu Ala Ala His His Trp Leu Asn Phe Leu Gln Ala Ala Tyr
               20                  25                  30
   cgc cta gaa ccc ggt ccc tcc agt tac gat ttc cac cag tta aaa aaa     144
   Arg Leu Glu Pro Gly Pro Ser Ser Tyr Asp Phe His Gln Leu Lys Lys
           35                  40                  45
   ttt ctt aaa ata gct tta gaa aca ccg gct cgg atc tgt ccc att aac     192
   Phe Leu Lys Ile Ala Leu Glu Thr Pro Ala Arg Ile Cys Pro Ile Asn
       50                  55                  60
   tac tcc ctc cta gcc agc cta ctc cca aaa gga tac ccc ggc cgg gtg     240
   Tyr Ser Leu Leu Ala Ser Leu Leu Pro Lys Gly Tyr Pro Gly Arg Val
   65                  70                  75                  80
   aat gaa att tta cac ata ctc atc caa acc caa gcc cag atc ccg tcc     288
   Asn Glu Ile Leu His Ile Leu Ile Gln Thr Gln Ala Gln Ile Pro Ser
                   85                  90                  95
   cgt ccc gcg cca ccg ccg ccg tca tcc ccc acc cac gac ccc ccg gat     336
   Arg Pro Ala Pro Pro Pro Pro Ser Ser Pro Thr His Asp Pro Pro Asp
               100                 105                 110
   tct gat cca caa atc ccc cct ccc tat gtt gag cct acg gcc ccc caa     384
   Ser Asp Pro Gln Ile Pro Pro Pro Tyr Val Glu Pro Thr Ala Pro Gln
           115                 120                 125
   gtc ctt cca gtc atg cat cca cat ggt gct cct cct aac cat cgc cca     432
   Val Leu Pro Val Met His Pro His Gly Ala Pro Pro Asn His Arg Pro
       130                 135                 140
   tgg caa atg aaa gac cta cag gcc att aag caa gaa gtc tcc caa gca     480
   Trp Gln Met Lys Asp Leu Gln Ala Ile Lys Gln Glu Val Ser Gln Ala
   145                 150                 155                 160
   gcc cct ggg agc ccc cag ttt atg cag acc atc cgg ctt gcg gtg cag     528
   Ala Pro Gly Ser Pro Gln Phe Met Gln Thr Ile Arg Leu Ala Val Gln
                   165                 170                 175
   cag ttt gac ccc act gcc aaa gac ctc caa gac ctc cta cag cac ccg     576
   Gln Phe Asp Pro Thr Ala Lys Asp Leu Gln Asp Leu Leu Gln His Pro
               180                 185                 190
   gta ttc cca ggc ggt ctc cca tcc aag tac taa                         609
   Val Phe Pro Gly Gly Leu Pro Ser Lys Tyr
           195                 200
<210> 27
   <211> 202
   <212> PRT
   <213> Human T-cell lymphotropic virus
<400> 27
   Met Gly Gln Ile Phe Ser Arg Ser Ala Ser Pro Ile Pro Arg Pro Pro
   1               5                   10                  15
   Arg Gly Leu Ala Ala His His Trp Leu Asn Phe Leu Gln Ala Ala Tyr
               20                  25                  30
   Arg Leu Glu Pro Gly Pro Ser Ser Tyr Asp Phe His Gln Leu Lys Lys
           35                  40                  45
   Phe Leu Lys Ile Ala Leu Glu Thr Pro Ala Arg Ile Cys Pro Ile Asn
       50                  55                  60
   Tyr Ser Leu Leu Ala Ser Leu Leu Pro Lys Gly Tyr Pro Gly Arg Val
   65                  70                  75                  80
   Asn Glu Ile Leu His Ile Leu Ile Gln Thr Gln Ala Gln Ile Pro Ser
                   85                  90                  95
   Arg Pro Ala Pro Pro Pro Pro Ser Ser Pro Thr His Asp Pro Pro Asp
               100                 105                 110
   Ser Asp Pro Gln Ile Pro Pro Pro Tyr Val Glu Pro Thr Ala Pro Gln
           115                 120                 125
   Val Leu Pro Val Met His Pro His Gly Ala Pro Pro Asn His Arg Pro
       130                 135                 140
   Trp Gln Met Lys Asp Leu Gln Ala Ile Lys Gln Glu Val Ser Gln Ala
   145                 150                 155                 160
   Ala Pro Gly Ser Pro Gln Phe Met Gln Thr Ile Arg Leu Ala Val Gln
                   165                 170                 175
   Gln Phe Asp Pro Thr Ala Lys Asp Leu Gln Asp Leu Leu Gln His Pro
               180                 185                 190
   Val Phe Pro Gly Gly Leu Pro Ser Lys Tyr
           195                 200

## Description

### TECHNICAL FIELD

The present invention relates to a method for reducing non-specific reaction in an immunoassay for anti-HTLV antibody in a sample using a modified p19 peptide and the use of the modified p19 peptide as a non-specific reaction-reducing agent in an immunoassay, whereby the non-specific reaction-reducing agent can be provided as part of an immunoassay kit.

### BACKGROUND ART

HTLV (human T cell leukemia virus, or human T-lymphotropic virus) is a retrovirus which infects T lymphocytes. HTLV Type 1 to Type 4 have been reported so far. Among these, Type 1 (HTLV-1) is known as a causative virus of adult T-cell leukemia, and it is said that about 0.3% of HTLV-1-antibody-positive individuals develop this disease. Although the frequency of its development is not high, an HTLV-1 antibody test is clinically important.

p19, which is one of the gag proteins of HTLV, has high immunogenicity among the constituents of the virus, and has been thought to be a useful antigen component for HTLV antibody tests. In particular, the C-terminal region (aa 120-130) of p19 has the highest reactivity (Non-patent Document 1), and a method in which a C-terminal fragment of p19 is used as an antigen for detecting anti-HTLV-1 antibody is known (Patent Document 1).

However, it has been pointed out that p 19 may also be involved in non-specific reaction which may cause false positives in HTLV antibody tests. Moreover, it has been pointed out that p 19 has cross reactivity with other viruses (Non-patent Document 2). Under such circumstances, use of p19 as an antigen for detecting the antibody needs to be restricted. In HTLV-1 antibody test kits that are commercially available at present, native antigens (that is, viral proteins extracted from HTLV-1-infected cells) are used.

Le Blanc et al (J.Virol 76(19) (2002) 10024-10029) describes how L domains in the Gag proteins of retroviruses are required for virus production and that mutations of the core motif cause a significant reduction in virus production.

Li et al., UniProt Accession No. Q2VA24 provides a nucleotide sequence for HTLV-1.

Mahieux et al., (J.Virol. 71(8) (1997) 6253-6258) describe the molecular characterisation and analyses of Simian T-Cell Lymphotrophic Virus Type 1.

### PRIOR ART DOCUMENT(S)

### PATENT DOCUMENT(S)

Patent Document 1: JP 3506252 B

### NON-PATENT DOCUMENT(S)

Non-patent Document 1: Palker et al., J Immunol. vol.136, p.2393-2397, 1986
Non-patent Document 2: Sato et al., J Gen Virol. vol.73, p.2969-2973, 1992

### SUMMARY OF THE INVENTION

### PROBLEMS TO BE SOLVED BY THE INVENTION

An object of the present invention is to provide a means for enabling reduction of non-specific reaction caused by p19 while a decrease in the measurement value is suppressed as much as possible in cases of HTLV antibody positive.

### MEANS FOR SOLVING THE PROBLEMS

As a result of intensive study, the present inventors discovered that the non-specific reaction caused by p19 is due to the primary amino acid sequence of p19, and that the responsible sequence for the non-specific reaction is the region of the 112th to 118th amino acids of p19. The present inventors also discovered that, by adding a p19 fragment having a substitution mutation in this region to an HTLV antibody assay system, the non-specific reaction can be largely reduced while a decrease in the reactivity with positive samples is suppressed as much as possible, thereby completing the present invention.

That is, the present invention provides a method for reducing non-specific reaction in an immunoassay for anti-HTLV antibody, said method comprising allowing a modified p19 peptide(s) to be present in an antigen-antibody reaction system, wherein said modified p19 peptide is a p19 fragment comprising the region of the 103rd to 130th amino acids in HTLV p19 and has at least one substitution mutation in the region corresponding to the 112th to 118th amino acids in p19. Also described is an immunoassay reagent for anti-HTLV antibody, comprising a modified p19 peptide(s), wherein said modified p19 peptide is a polypeptide which is constituted by a p19 fragment comprising the region of the 103rd to 130th amino acids in HTLV p19 and has at least one substitution mutation in the region corresponding to the 112th to 118th amino acids in p19. The present invention further provides the use of a modified p19 peptide(s), wherein said modified p19 peptide is a p19 fragment comprising the region of the 103rd to 130th amino acids in HTLV p19 and has at least one substitution mutation in the region corresponding to the 112th to 118th amino acids in p19 as a non-specific reducing agent for an immunoassay for anti-HTLV antibody, optionally whereby the non-specific reaction-reducing agent is provided as part of an immunoassay kit. Also described is an artificial polypeptide which is constituted by a p19 fragment comprising the region of the 103rd to 130th amino acids in HTLV p19 and has at least one substitution mutation in the region corresponding to the 112th to 118th amino acids in p19.

### EFFECT OF THE INVENTION

The present invention provided a means which enables desirable reduction of non-specific reaction which is problematic in HTLV antibody tests by an immunoassay. By allowing a modified p19 peptide(s) having an amino acid substitution in a particular region to be present in an antigen-antibody reaction system, the non-specific reaction can be reduced while suppressing a decrease in the measurement value of positive samples. Since the non-specific reaction may cause false positives, reduction of the non-specific reaction allows reduction of occurrence of false positives, by which the reliability of HTLV antibody test kits can be increased. Moreover, since the present invention enables use of highly immunogenic p19 as an antigen, the sensitivities of HTLV antibody test kits can be improved.

### MODE FOR CARRYING OUT THE INVENTION

p19 is one of the three kinds of proteins generated by cleavage of the gag precursor protein expressed from the *gag* gene of HTLV, which cleavage is catalyzed by the proteolytic enzyme encoded by the *prt* gene. SEQ ID NOs:26 and 27 are examples of the base sequence of the HTLV-1 *gag* gene and the amino acid sequence of the gag precursor protein encoded thereby, and deposited in GenBank under Accession No. D14335. The region of the 1st to 130th amino acids in SEQ ID NO:27 corresponds to p19.

In the present invention, HTLV is typically HTLV-1.

"Modified p19 peptide" in the present invention is a polypeptide which is a p19 fragment comprising the region (SEQ ID NO:1) of the 103rd to 130th amino acids in HTLV-1 p19, and has at least one substitution mutation in the region of seven residues corresponding to the 1 12th to 118th amino acids in p19 (the 10th to 16th amino acids in SEQ ID NO:1). The modified p19 peptide is typically an artificial polypeptide. In the modified p19 peptide, the substitution mutation may be substitution with an amino acid which is different from the amino acid residue in the wild-type sequence (or native sequence), and does not need to be substitution with a residue having a property largely different from that of the original residue (in terms of, for example, whether the side chain is hydrophilic or not; whether the amino acid is neutral amino acid, basic amino acid, or acidic amino acid; and/or whether the amino acid has an aromatic side chain or not).

In the present invention, "polypeptide" and "peptide" have the same meaning, and both of these mean a molecule formed by binding of two or more amino acid residues to each other via peptide bonds.

The terms "artificial polypeptide" and "artificial peptide" mean a polypeptide which is artificially produced by a chemical synthesis, genetic engineering technique or any other method, and does not include one which is obtained by collecting a protein expressed and produced from HTLV genome in cells infected with HTLV.

In the modified p19 peptide, one or more (for example, about one to several) amino acid residues which are absent in the native p19 sequence may be added to one or both of the termini of the peptide. Usually, such an addition of one or more residues may be carried out for convenience in the peptide production process, or for convenience in immobilization of the peptide on a solid support. The term "constituted by a p19 fragment" encompasses a peptide having such an addition of one or more amino acid residues. Similarly, the term "constituted by an amino acid sequence" encompasses a peptide comprising the amino acid referred to and such an addition of one or more amino acids described above.

The above-described substitution mutation(s) in the region of seven residues contained in the modified p19 peptide may be substitution of any residue(s) among the seven residues. Only one residue may be substituted, or two or more residues may be substituted. Substitution of any residue(s) in the region of the seven residues allows about 50% or more reduction of the non-specific reaction that occurs in the immunoassay in which a native antigen is used (see Examples below). Modified p19 peptides having the amino acid sequences shown in SEQ ID NOs:2 to 8 are those in which one residue in the region of the seven residues is substituted with alanine. These peptides are examples of modified p19 peptides which can be used in the present invention.

The modified p19 peptide preferably comprises substitution of at least one residue of the 114th amino acid and the 117th amino acid in p19 (the 12th amino acid and the 15th amino acid in SEQ ID NO:1, respectively). Specific examples of such a modified p19 peptide include, but are not limited to, modified p19 peptides having the amino acid sequences of SEQ ID NO:9 to 15 (for example, polypeptides composed of the amino acid sequences of SEQ ID NOs:17 to 25, in each of which Lys is added to the N-terminus of a modified p19 partial sequence).

In the method of the present invention, a modified p19 peptide(s) is/are allowed to be present in a system for antigen-antibody reaction between anti-HTLV antibody in a sample and an antigen. As the HTLV antigen, only the modified p19 peptide(s) may be used, or both the modified p19 peptide(s) and at least one of other HTLV antigens may be used. "Other HTLV antigens" each may be a native antigen, or an artificial peptide antigen designed based on the amino acid sequence of an HTLV protein. Examples of the "artificial peptide antigen" herein include p19 proteins having native sequences and partial peptides thereof; and HTLV proteins other than p19 (for example, gp21, gp46, p24, and p15) having native or modified sequences, and partial peptides thereof. In cases where a native antigen is used, a native antigen containing native p19 may be used in a mixture with the modified p19 peptide(s), or a native antigen from which the p19 fraction is removed may be used in a mixture with the modified p19 peptide(s).

The sample may be the same as a sample to be used in a common HTLV antibody test, and is usually a blood sample such as whole blood, plasma, or serum. The sample is diluted as appropriate before use.

In an immunoassay of antibody, an antigen immobilized on a solid support is usually used for convenience in B/F separation. The modified p19 peptide(s) can be used in a state where the peptide(s) is/are immobilized on such a solid support. In cases where both the modified p19 peptide(s) and at least one of other HTLV antigens are used, the modified p19 peptide(s) may be used in a state where the modified p19 peptide(s) is/are immobilized in combination with the at least one of other HTLV antigens. In cases where the solid support is particles such as magnetic particles or latex particles, the term "immobilized in combination" includes concurrently immobilizing the modified p19 peptide(s) and the other HTLV antigen(s) on the particles, and separately immobilizing the modified p19 peptide(s) and the other HTLV antigen(s) on the particles and then mixing the peptide- and antigen-immobilized particles together.

The solid support is not limited, and may be a solid support conventionally used for immunoassays. Examples of the solid support include plastic plates, minute particles, and fibrous substances. The materials of these supports are not limited, and conventionally known materials may be used. Examples of the minute particles include glass beads; various plastic beads such as polystyrene beads; latex particles; gelatin particles; and various ferrite particles (for example, see JP H03-115862 A). Examples of the fibrous substances include those comprising cellulose, nitrocellulose, chitosan, polyethylene glycol polymers, and/or silane polymers, as constituents.

Immobilization of the modified p19 peptide(s), and at least one of other HTLV antigens if used, on such a support may be carried out using a known method such as physical adsorption or chemical bonding using a cross-linking agent, as appropriate.

The modified p19 peptide(s) may also be used in a form in which the peptide(s) is/are labeled with a labeling substance as described later. Also in such a case, a mixture of the modified p19 peptide(s) and at least one of other HTLV antigens may be used as a labeled antigen.

Antibody measurement methods *per se* are well known in the art. When classified based on the reaction mode, immunoassays can be classified into sandwich methods, competition methods, agglutination methods, immunochromatography, Western blotting or the like; and, when classified based on the label, immunoassays can be classified into radioimmunoassays, fluoroimmunoassays, enzyme immunoassays (EIA), biotin immunoassays or the like. Specific examples of the techniques for antibody testing in which an antigen is used include, but not limited to, EIA (such as ELISA, CLEIA (chemiluminescent enzyme immunoassay), Western blotting), chemiluminescent immunoassay (CLIA), agglutination methods (such as a latex agglutination method), complement-fixation reactions (CF) and the like. The immunoassay for anti-HTLV antibody to which the present invention is applied may be carried out by any method as long as anti-HTLV antibody in a sample can be specifically measured by the method. It is noted here that, in the present invention, the term "measure/measurement" includes detection, quantification and semi-quantification.

For example, in the case of ELISA or CLEIA, the modified p19 peptide(s) is/are combined with, if desired, at least one of other HTLV antigens and immobilized on a solid support such as a plate or particles (sensitization), and a sample is reacted therewith to capture anti-HTLV antibody in the sample on the support. The solid support is then washed, and reacted with a labeled anti-immunoglobulin antibody or a labeled HTLV antigen. After washing the solid support again, a signal from the labeling substance is measured. In the case where the labeling substance is an enzyme, a substrate substance for the enzyme may be added, and the level of the enzyme reaction (the amount of luminescence, coloration, or the like) may be measured. In the case where biotin or hapten is used as the labeling substance, streptavidin or a hapten antibody to which an enzyme, fluorescent substance, chemiluminescent substance, staining substance, radioactive substance or the like is bound may be used. Based on the signal measurement value, the anti-HTLV antibody captured on the support can be measured.

In the case of Western blotting, for example, the modified p19 peptide(s) or a mixture of the modified p19 peptide(s) and at least one of other HTVL antigens may be subjected to electrophoresis, and then transferred onto a membrane. The membrane may then be reacted with a sample, and subsequently with a labeled anti-immunoglobulin antibody or a labeled HTLV antigen.

In the case of an agglutination method, for example, the modified p19 peptide(s) may be combined with, if desired, at least one of other HTLV antigens, and immobilized on gelatin particles, latex particles or the like. A sample may then be reacted therewith, and the amount of agglutination of the particles may be measured based on the absorbance or the like.

In cases where a labeled anti-immunoglobulin antibody is used, the type of the anti-immunoglobulin antibody is not limited since the subclass of the anti-HTLV antibody to be measured is not limited. For example, one of an anti-IgG antibody and an anti-IgM antibody, or a mixture of both of these, may be used. Examples of the HTLV antigen which may be used with a label include modified p19 peptides, and other HTLV antigens as defined above. A mixture of a plurality of these antigens may be used as the labeled HTLV antigen. In cases where the immunoassay is carried out using a labeled HTLV antigen, the modified p19 peptide(s) may be used for at least one of the solid-phase antigen to be immobilized on the support (the antigen to be transferred onto the membrane, in the case of Western blotting) and the labeled HTLV antigen.

The modified p19 peptide can be produced by a conventional chemical synthesis method. Specific examples of the chemical synthesis method include the Fmoc method (fluorenylmethyloxycarbonyl method), the tBoc method (*t-*butyloxycarbonyl method) and the like. The modified p19 peptide can also be synthesized by a conventional method using any of various types of commercially available peptide synthesizers.

The modified p19 peptide can also be produced by a conventional genetic engineering technique. As described above, the p19 amino acid sequence and the base sequence encoding it are known as deposited in the database, and described also in SEQUENCE LISTING of the present application. Further, since the codons encoding each amino acid are known, the base sequence of a polynucleotide encoding a specific amino acid sequence can be easily specified. Therefore, even in the case of a modified p19 peptide whose sequence is different from the sequence of wild-type p19, the base sequence of a polynucleotide encoding such a modified p19 peptide can be easily specified, and hence production of the modified p19 peptide by a genetic engineering technique is also possible.

Briefly, the production process by the genetic engineering technique is as follows. First, a polynucleotide encoding a desired modified p19 peptide is prepared using a commercially available nucleic acid synthesizer, or by appropriately introducing a mutation(s) by a known method into DNA amplified from the wild-type HTLV genome by PCR. The polynucleotide is then incorporated into an appropriate expression vector, and expression of the polypeptide is carried out using an appropriate expression system. By recovering and purifying the expressed polypeptide, the modified p19 peptide of interest can be obtained as a recombinant polypeptide.

Since the modified p19 peptide itself can be used as an antigen for measuring anti-HTLV antibody, the modified p19 peptide can be provided as an immunoassay reagent. The immunoassay reagent may comprise only the modified p19 peptide(s) in an appropriate buffer, or may further comprise at least one of other HTLV antigens. The immunoassay reagent may further comprise one or more of other components useful for, for example, stabilization of the modified p19 peptide(s) and/or the like. In cases where the immunoassay reagent further comprises at least one of other HTLV antigens, the modified p19 peptide(s) and other HTLV antigen(s) may be packaged in separate containers, or may be preliminarily mixed together and packaged in a single container. The immunoassay reagent may be in a form in which the modified p19 peptide(s) is/are immobilized on a solid support.

The modified p19 peptide can be provided as a non-specific reaction-reducing agent for an immunoassay for anti-HTLV antibody. The non-specific reaction-reducing agent comprises the modified p19 peptide(s), and may further comprise one or more of other components useful for, for example, stabilization of the peptide. The non-specific reaction-reducing agent may be used by addition to the antigen-antibody reaction system in an immunoassay for anti-HTLV antibody. For example, the non-specific reaction-reducing agent may be added to an HTLV antigen to carry out the reaction with a sample. Or, the non-specific reaction-reducing agent may be combined with an HTLV antigen and immobilized on a solid support. Or, the non-specific reaction-reducing agent may be in a labeled form for use as a labeled antigen. By allowing the non-specific reaction-reducing agent to coexist in the antigen-antibody reaction system in an immunoassay for anti-HTLV antibody, the non-specific reaction can be reduced, and occurrence of false positives can be suppressed.

The immunoassay reagent or the non-specific reaction-reducing agent described above may be combined with one or more of other reagents and the like as appropriate, and provided as an immunoassay kit for anti-HTLV antibody in a sample. Other reagents necessary for an immunoassay are well known. The kit may comprise, for example, a sample dilution liquid and/or washing liquid, in addition to the immunoassay reagent or the non-specific reaction-reducing agent described above. In cases where the labeling substance used for the labeled antibody is an enzyme, the kit may also comprise a substrate liquid for the enzyme, and/or the like.

The artificial polypeptide described herein is a polypeptide preferably used as the modified p19 peptide described above. The artificial polypeptide is a p19 fragment comprising the region of the 103rd to 130th amino acids in HTLV-1 p19 (SEQ ID NO:1), and has at least one substitution mutation in the region of seven residues corresponding to the 112th to 118th amino acids in p19 (the 10th to 16th amino acids in SEQ ID NO:1). Preferred modes of the artificial polypeptide are the same as those described for the modified p19 peptide.

### EXAMPLES

The present invention is described below concretely by way of Examples. However, the present invention is not limited to the Examples below.

### 1. Search for Non-specificity-responsible sequence in p19 in HTLV-1 Antibody Test

It is known that the C-terminal region of p19 (aa103-130) can be used as an antigen in an HTLV-1 antibody test. In order to identify a region important for absorbing non-specific reaction in this region, the p19 fragments shown in Table 1 were prepared by chemical synthesis, and tests for absorption of non-specific reaction were carried out using non-specific samples which had been confirmed to cause non-specific reaction.

To a suspension of particles sensitized with an HTLV virus native antigen, each of the peptides shown in Table 1 was added at 10 µg/mL or 100 µg/mL, and the reactivities of the peptide with two non-specific samples and one low-level positive sample were observed. As a negative control (N. C.), HTLV-1-negative delipidated serum was used. As a positive control (P. C.), HTLV-1-positive serum (diluted with a negative control liquid) was used. The actual measurement was carried out using a commercially available fully automated chemiluminescent enzyme immunoassay system "LUMIPULSE" (registered trademark) (manufactured by Fujirebio Inc.). Each sample was similarly subjected to measurement under conditions where no peptide was added, to calculate a reference measurement value (reference count value). The effect to suppress non-specific reaction was evaluated based on the ratio (%) to the reference count value.

**[Table 1]**

| Peptide No. | | Position in p19 protein |
|---|---|---|
| 7 | PSSPTHDPPDSDPQIPPPYVEPTAPQVL | aa103-130 |
| 13 | PTHDPPDSDPQIPPPYVEPTAPQVL | aa106-130 |
| 14 | DPPDSDPQIPPPYVEPTAPQVL | aa109-130 |
| 15 | DSDPQIPPPYVEPTAPQVL | aa112-130 |
| 16 | PQIPPPYVEPTAPQVL | aa115-130 |
| 17 | PPPYVEPTAPQVL | aa118-130 |
| 18 | YVEPTAPQVL | aa121-130 |
| 19 | PSSPTHDPPDSDPQIPPPYVEPTAP | aa103-127 |
| 20 | PSSPTHDPPDSDPQIPPPYVEP | aa103-124 |
| 21 | PSSPTHDPPDSDPQIPPPY | aa103-121 |
| 22 | PSSPTHDPPDSDPQIP | aa103-118 |
| 23 | PSSPTHDPPDSDP | aa103-115 |
| 24 | PSSPTHDPPD | aa103-112 |

As a result, it was confirmed that the non-specific reaction decreased in the cases where any of the peptides Nos. 13 to 15 and Nos. 19 to 22 was added to the particles before the measurement (Table 2). In particular, the peptides Nos. 20 to 22 showed good results; they largely lowered the count for the non-specific sample No. 0045 while hardly lowering the count for the low-level positive sample No. 53. From these results, it was thought that the amino acid region of aa 112-118 (this region is hereinafter referred to as "non-specificity-responsible sequence") is essential for decreasing the non-specific reaction.

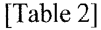

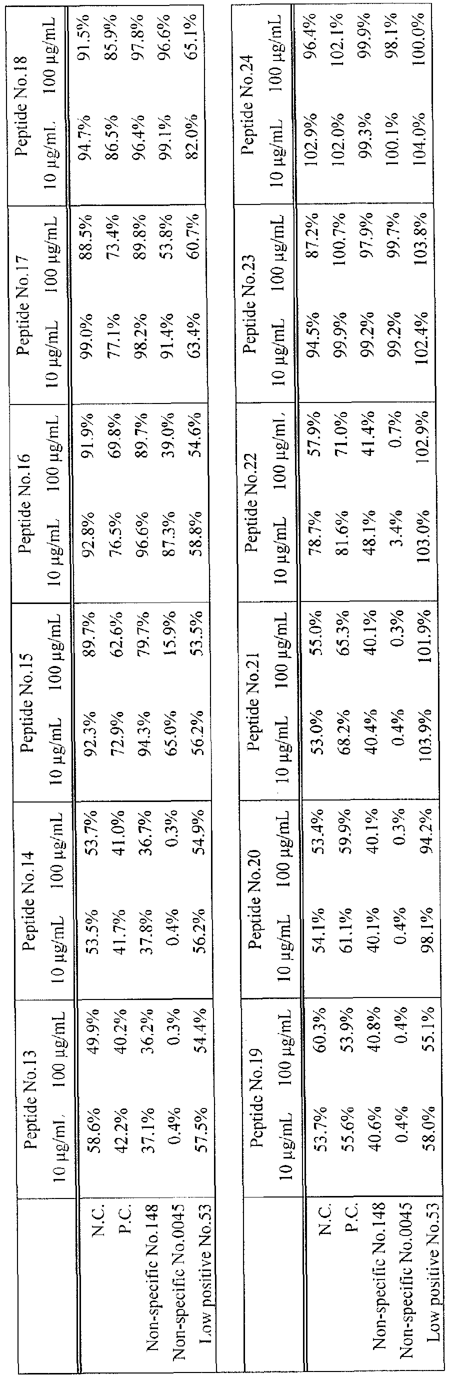

### 2. Absorption Test Using Modified p19 Peptides Having Substitution Mutations in Non-specificity-responsible Sequence

Modified p19 peptides (Table 3) were prepared by substituting any one of the residues in the non-specificity-responsible sequence with alanine one by one, and non-specificity absorption tests were carried out using the modified p19 peptides in the same manner as described above.

**[Table 3]**

| Peptide No. | | |
|---|---|---|
| 7 | PSSPTHDPPDSDPQIPPPYVEPTAPQVL | SEQ ID NO: 1 |
| 34 | PSSPTHDPP**A**SDPQIPPPYVEPTAPQVL | SEQ ID NO: 2 |
| 35 | PSSPTHDPPD**A**DPQIPPPYVEPTAPQVL | SEQ ID NO: 3 |
| 36 | PSSPTHDPPDS**A**PQIPPPYVEPTAPQVL | SEQ ID NO: 4 |
| 37 | PSSPTHDPPDSD**A**QIPPPYVEPTAPQVL | SEQ ID NO: 5 |
| 38 | PSSPTHDPPDSDP**A**IPPPYVEPTAPQVL | SEQ ID NO: 6 |
| 39 | PSSPTHDPPDSDPQ**A**PPPYVEPTAPQVL | SEQ ID NO: 7 |
| 40 | PSSPTHDPPDSDPQI**A**PPYVEPTAPQVL | SEQ ID NO: 8 |

The results are shown in Table 4. The non-specific reaction could be reduced by 50% or more by addition of any of the modified peptides at 100 µg/mL. In particular, the peptides No. 36 and No. 39 showed smaller decreases in the measured value of the positive control, compared to other peptides.

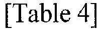

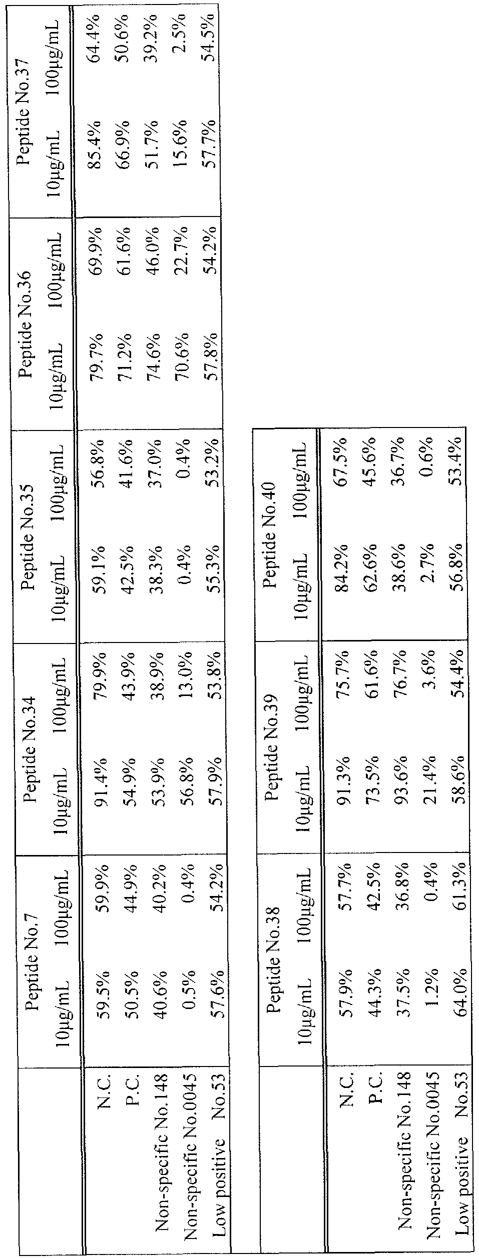

### 3. Study on Reactivities of Modified p19 Peptides with Non-specific Samples

The peptides No. 36 and No. 39, which showed smaller decreases in the measured value of the positive control, were selected for the present study. Mutant peptides were further prepared as shown in Table 5 below (K was added to the N-terminus of each peptide in order to immobilize the peptide onto carboxylated particles through its amino group).

By the following procedure, magnetic particles were sensitized with each of the modified p19 peptides in Table 5, to prepare modified-p19-peptide-sensitized particles.
(1) With Milli-Q water, 15 mg of carboxylated magnetic particles (manufactured by Fujirebio Inc.) were washed three times.
(2) To the washed particles, 4 mg of EDC (1-ethyl-3-(3-dimethylaminopropyl)-carbodiimide hydrochloride) was added, and the resulting mixture was incubated with gentle stirring at 25°C for 30 minutes.
(3) To the mixture, 7.5 mg of a modified p19 peptide was added, and the resulting mixture was incubated with gentle stirring at 25°C for 60 minutes.
(4) The particles were washed three times with 2% BSA/Tris (pH 7.2), to provide modified-p 19-peptide-sensitized particles.

Using the modified-p19-peptide-sensitized particles, reactivities with eight non-specific samples were observed. The measurement operation was carried out using a commercially available fully automated chemiluminescent enzyme immunoassay system "LUMIPULSE" (registered trademark) (manufactured by Fujirebio Inc.).

**[Table 5]**

| | | |
|---|---|---|
| K + Peptide 7 (p19 aa103-130) | KPSSPTHDPPDSDPQIPPPYVEPTAPQVL | SEQ ID NO: 16 |
| K + Peptide 36 (p19 aal 03-130, D114A) | KPSSPTHDPPDS**A**PQIPPPYVEPTAPQVL | SEQ ID NO: 17 |
| K + Peptide 39 (p19 aa103-130, I117A) | KPSSPTHDPPDSDPQ**A**PPPYVEPTAPQVL | SEQ ID NO: 18 |
| K + Peptide 41 (p19 aa103-130, D114A & I117A) | KPSSPTHDPPDS**A**PQ**A**PPPYVEPTAPQVL | SEQ ID NO: 19 |
| K + Peptide 42 (p19 aa103-130, D114N) | KPSSPTHDPPDS**N**PQIPPPYVEPTAPQVL | SEQ ID NO: 20 |
| K + Peptide 43 (p19 aa103-130, I117L) | KPSSPTHDPPDSDPQ**L**PPPYVEPTAPQVL | SEQ ID NO: 21 |
| K + Peptide 44 (p19 aa103-130, D114N & I117L) | KPSSPTHDPPDS**N**PQ**L**PPPYVEPTAPQVL | SEQ ID NO: 22 |
| K + Peptide 45 (p19 aa103-130, D114E) | KPSSPTHDPPDS**E**PQIPPPYVEPTAPQVL | SEQ ID NO: 23 |
| K + Peptide 46 (p19 aa103-130, D114N & I117E) | KPSSPTHDPPDS**N**PQ**E**PPPYVEPTAPQVL | SEQ ID NO: 24 |
| K + Peptide 47 (p19 aa103-130, D114E & I117E) | KPSSPTHDPPDS**E**PQ**E**PPPYVEPTAPQVL | SEQ ID NO: 25 |

The sequences of Peptides 41 to 47 are shown in SEQ ID NOS: 9 to 15, respectively.

The results are shown in Table 6. All mutant peptides could be confirmed to have an effect to suppress the non-specific reaction to not more than about 50% in all non-specific samples used, without largely lowering the measured value of the positive control.

**[Table 6]**

| Ratio (%) of the counts to the count of p19 peptide No. 7 | | | | | | | | | | |
|---|---|---|---|---|---|---|---|---|---|---|
| | No. 7 | No. 36 | No. 39 | No. 41 | No. 42 | No. 43 | No. 44 | No. 45 | No. 46 | No. 47 |
| Negative Control | 100 | 81.9 | 82.6 | 85.8 | 83.9 | 87.7 | 84.5 | 78.7 | 80.6 | 85.8 |
| Positive Control | 100 | 64.7 | 46.9 | 63 | 61 | 53.7 | 56.2 | 38.9 | 67.4 | 59.1 |
| Non-specific sample 1 | 100 | 3.9 | 3.7 | 3.7 | 4 | 6.7 | 3.7 | 4.1 | 3 | 3.3 |
| Non-specific sample 2 | 100 | 26.4 | 26.2 | 27.2 | 32.5 | 33.8 | 25 | 25.7 | 26.7 | 26 |
| Non-specific sample 3 | 100 | 46.5 | 47.9 | 47.1 | 41 | 49.3 | 45.2 | 45.4 | 41 | 39.6 |
| Non-specific sample 4 | 100 | 4.2 | 3.9 | 3.8 | 4.5 | 5.8 | 4 | 3.9 | 3.6 | 3.5 |
| Non-specific sample 5 | 100 | 15.8 | 13.7 | 18.1 | 13.6 | 13.5 | 13.9 | 13.8 | 15.6 | 15.4 |
| Non-specific sample 6 | 100 | 51.6 | 51.9 | 49 | 44.5 | 52.2 | 51.3 | 51.6 | 42.7 | 46.6 |
| Non-specific sample 7 | 100 | 36.6 | 38.1 | 38.1 | 33.8 | 146.1 | 38.7 | 37.9 | 35.1 | 37.4 |
| Non-specific sample 8 | 100 | 0.6 | 0.5 | 0.5 | 0.8 | 0.8 | 0.5 | 0.5 | 0.4 | 0.4 |

## Claims

1. A method for reducing non-specific reaction in an immunoassay for anti-HTLV antibody, said method comprising allowing a modified p19 peptide(s) to be present in an antigen-antibody reaction system, wherein the modified p19 peptide is a p19 fragment comprising the region of the 103rd to 130th amino acids in HTLV p19 and has at least one substitution mutation in the region corresponding to the 112th to 118th amino acids in p19.

2. The method according to claim 1, wherein the modified p19 peptide comprises substitution mutation of at least one of the amino acid residues corresponding to the 114th amino acid and the 117th amino acid in p19.

3. The method according to claim 1, wherein the modified p19 peptide is a polypeptide comprising the amino acid sequence of any one of SEQ ID NOs:2 to 15.

4. The method according to claim 2, wherein the modified p19 peptide is a polypeptide comprising the amino acid sequence of any one of SEQ ID NOs:17 to 25.

5. The method according to any one of claims 1 to 4, wherein said modified p19 peptide(s) is/are immobilized on a solid support.

6. Use of a modified p19 peptide(s), wherein the modified p19 peptide is a p19 fragment comprising the region of the 103rd to 130th amino acids in HTLV p19 and has at least one substitution mutation in the region corresponding to the 112th to 118th amino acids in p19, as a non-specific reaction-reducing agent for an immunoassay for anti-HTLV antibody.

7. The use of claim 6 whereby the non-specific reaction-reducing agent is provided as part of an immunoassay kit.

8. The use of claims 6 or 7 wherein the modified polypeptide comprises substitution of at least one of the amino acid residues corresponding to the 114th amino acid and the 117th amino acid in p19.

9. The use of claims 6 tor 7 wherein the modified polypeptide comprises the amino acid sequence of any one of SEQ ID NOs:2 to 15.

10. The use of claim 8 wherein the modified polypeptide comprises the amino acid sequence of any one of SEQ ID NOs:17 to 25.

## Patentansprüche

1. Verfahren zur Verminderung einer nicht spezifischen Reaktion in einem Immuntest für Anti-HTLV-Antikörper, wobei das Verfahren das Zulassen des Vorhandenseins von modifiziertem/n p19-Peptid(en) in einem Antigen-Antikörper-Reaktionssystem umfasst, wobei das modifizierte p19-Peptid ein p19-Fragment ist, das den Bereich der 103. bis 130. Aminosäure in HTLV-p19 umfasst und zumindest eine Substitutionsmutation in der Region aufweist, die der 112. bis 118. Aminosäure in p19 entspricht.

2. Verfahren nach Anspruch 1, wobei das modifizierte p19-Peptid eine Substitutionsmutation von zumindest einem der Aminosäurereste umfasst, die der 114. Aminosäure und der 117. Aminosäure in p19 entsprechen.

3. Verfahren nach Anspruch 1, wobei das modifizierte p19-Peptid ein Polypeptid ist, das die Aminosäuresequenz von einer beliebigen der SEQ ID NO: 2 bis 15 umfasst.

4. Verfahren nach Anspruch 2, wobei das modifizierte p19-Peptid ein Polypeptid ist, das die Aminosäuresequenz von einer beliebigen der SEQ ID NO: 17 bis 25 umfasst.

5. Verfahren nach einem der Ansprüche 1 bis 4, wobei das/die modifizierte/n p19-Peptid/e auf einem festen Träger immobilisiert ist/sind.

6. Verwendung von modifiziertem/n p19-Peptid(en) als nicht spezifisches reaktionsverminderndes Mittel für einen Immuntest für Anti-HTLV-Antikörper, wobei das modifizierte p19-Peptid ein p19-Fragment ist, das den Bereich der 103. bis 130. Aminosäure in HTLV-p19 umfasst und zumindest eine Substitutionsmutation in der Region aufweist, die der 112. bis 118. Aminosäure in p19 entspricht.

7. Verwendung nach Anspruch 6, wobei das nicht spezifische reaktionsvermindernde Mittel als Teil eines Immuntestsets bereitgestellt wird.

8. Verwendung nach Anspruch 6 oder 7, wobei das modifizierte Polypeptid eine Substitution von zumindest einem der Aminosäurereste, die der 114. Aminosäure und der 117. Aminosäure in p19 entsprechen, umfasst.

9. Verwendung nach Anspruch 6 oder 7, wobei das modifizierte Polypeptid die Aminosäuresequenz aus einer beliebigen der SEQ ID NO: 2 bis 15 umfasst.

10. Verwendung nach Anspruch 8, wobei das modifizierte Polypeptid die Aminosäuresequenz aus einer beliebigen der SEQ ID NO: 17 bis 25 umfasst.

## Revendications

1. Procédé de réduction d'une réaction non spécifique dans un dosage immunologique d'anticorps anti-HTLV, ledit procédé consistant à permettre la présence d'un ou de peptide(s) p19 modifié(s) dans un système de réaction antigène-anticorps, dans lequel le peptide p19 modifié est un fragment de p19 comprenant la région des 103ème à 130ème acides aminés de p19 de HTLV et présente au moins une mutation de substitution dans la région correspondant aux 112ème à 118ème acides aminés de p19.

2. Procédé selon la revendication 1, dans lequel le peptide p19 modifié comprend une mutation de substitution d'au moins un des résidus d'acide aminé correspondant au 114ème acide aminé et au 117ème acide aminé de p19.

3. Procédé selon la revendication 1, dans lequel le peptide p19 modifié est un polypeptide comprenant la séquence d'acides aminés de l'une quelconque des SEQ ID NO:2 à 15.

4. Procédé selon la revendication 2, dans lequel le peptide p19 modifié est un polypeptide comprenant la séquence d'acides aminés de l'une quelconque des SEQ ID NO:17 à 25.

5. Procédé selon l'une quelconque des revendications 1 à 4, dans lequel ledit ou lesdit) peptide(s) p19 modifié(s) est/sont immobilisé(s) sur un support solide.

6. Utilisation d'un ou plusieurs peptides p19 modifiés, dans laquelle le peptide p19 modifié est un fragment de p19 comprenant la région des 103ème à 130ème acides aminés de p19 de HTLV et présente au moins une mutation de substitution dans la région correspondant aux 112ème à 118ème acides aminés de p19, en tant qu'agent réducteur de réaction non spécifique pour un dosage immunologique d'immuno-anticorps anti-HTLV.

7. Utilisation selon la revendication 6, dans laquelle l'agent réducteur de la réaction non spécifique est fourni en tant que partie d'un kit de dosage immunologique.

8. Utilisation selon la revendication 6 ou 7, dans laquelle le polypeptide modifié comprend la substitution d'au moins l'un des résidus d'acide aminé correspondant au 114ème acide aminé et au 117ème acide aminé de p19.

9. Utilisation selon les revendications 6 à 7, dans laquelle le polypeptide modifié comprend la séquence d'acides aminés de l'une quelconque des SEQ ID NO:2 à 15.

10. Utilisation selon la revendication 8, dans laquelle le polypeptide modifié comprend la séquence d'acides aminés de l'une quelconque des SEQ ID NO:17 à 25.
